(19) Europäisches Patentamt / European Patent Office / Office européen des brevets

(11) **EP 3 586 731 A1**

(12) **EUROPEAN PATENT APPLICATION**

(43) Date of publication:
01.01.2020 Bulletin 2020/01

(51) Int Cl.:
*A61B 5/022* (2006.01)    *A61B 5/0225* (2006.01)
*A61B 5/0235* (2006.01)

(21) Application number: **18179799.4**

(22) Date of filing: **26.06.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **HILGERS, Achim**
  **5656 AE Eindhoven (NL)**
• **HILBIG, Rainer**
  **5656 AE Eindhoven (NL)**
• **SCHMITT, Lars**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **SYSTEM FOR USE WITH A WEARABLE CUFF**

(57) There is provided a system (100) for use with a wearable cuff (200) in measuring a physiological characteristic of a subject (300). The system (100) comprises a chamber (102) configured to hold a fluid (104) at a pressure that is greater than atmospheric pressure. The system (100) also comprises a valve and/or a micro pump (106) configured to couple the chamber (102) to the cuff (200). The valve and/or a micro pump (106) is operable to control fluid flow between the chamber (102) and the cuff (200) to increase the pressure in the cuff (200) to a predetermined threshold pressure and vary the pressure in the cuff (200) around the predetermined threshold pressure.

100

200

102

106

104

108

300

Fig. 1

EP 3 586 731 A1

**Description**

FIELD OF THE INVENTION

The disclosure relates to a system for use with a wearable cuff in measuring a physiological characteristic of a subject.

BACKGROUND OF THE INVENTION

The most basic functions of the body of a subject can be monitored by measuring their physiological characteristics (or vital signs). The physiological characteristics of a subject are particularly useful in detecting or monitoring medical problems. They can be measured at various locations, such as in a medical environment, at home, at a site of a medical emergency, or elsewhere, and may be measured by a medical professional (e.g. a general practitioner, doctor, nurse or health care provider) or the subject themselves. Next to body temperature and respiration rate (or rate of breathing), the main physiological characteristics that are routinely measured are pulse rate and blood pressure.

The pulse rate is a measurement of the heart rate, or the number of times the heart beats per minute. As the heart pushes blood through the arteries, the arteries expand and contract with the flow of the blood. Taking a pulse not only measures the heart rate, but also can indicate the heart rhythm and the strength of the pulse. Both the heart rhythm and the strength of the pulse may be important in the diagnosis of disease. The blood pressure is a measurement of the force of the blood pushing against artery walls. Each time the heart beats, it pumps blood into the arteries, resulting in the highest blood pressure as the heart contracts. Generally, when measuring the blood pressure, two numbers are recorded. The higher number is indicative of a systolic blood pressure (SBP), which corresponds to a pressure inside an artery when the heart contracts and pumps blood through the body. The lower number is indicative of a diastolic blood pressure (DBP), which corresponds to a pressure inside an artery when the heart is at rest and is filling with blood. Both the SBP and DBP are usually measured in millimeters of mercury (mmHg).

When measuring physiological characteristics of a subject, a wearable cuff is often used. The wearable cuff is placed around a part (e.g. an arm, wrist, or a digit) of the body of the subject and inflated to pressurize the wearable cuff. The physiological characteristics of the subject can either be measured during inflation of the wearable cuff or deflation of the wearable cuff. The blood pressure is an example of a physiological characteristic that is typically measured when using a wearable cuff or, more specifically, a blood pressure cuff, which is also referred to as a sphygmomanometer. The most widely used non-invasive methods for measuring blood pressure include an auscultatory method, where blood pressure is measured manually, and an oscillometric method, where blood pressure is measured automatically (e.g. electronically). In both methods, the wearable cuff is inflated at a pressure higher than the systolic pressure and then slowly deflated.

The auscultatory method is based on measuring sounds (which are known as Korotkoff sounds) created by the artery under the cuff, which appear when the cuff pressure is equal to the SBP and disappear when the remaining pressure is equal to the DBP. The measurement of the sounds is usually performed manually with a stethoscope that is placed over the artery just below the cuff. The oscillometric method is based on measuring (electromechanically) the pressure oscillations induced in the wearable cuff by each heart beat. The pressure oscillations appear when the pressure in the wearable cuff equals the SBP, are maximum at the mean blood pressure, and disappear at the DBP.

In the case of a smaller wearable cuff (e.g. for a finger), there exist systems comprising a wearable cuff having a built-in photoplethysmography (PPG) sensor and a pressure controller unit configured to be placed on the wrist of the subject. The PPG does not provide blood pressure values but rather represents changes in the blood volume in the artery under the wearable cuff, which correspond to changes in the pressure in the wearable cuff. Where the blood volume in the artery under the wearable cuff is constant, the blood pressure value can be determined as the pressure in the wearable cuff. Therefore, the continuously changing pressure in the wearable cuff, which is applied from the outside of the finger, corresponds to the intra-arterial pressure and can thus be used as an instantaneous, continuous measure for arterial blood pressure.

When using a wearable cuff to measure physiological characteristics of a subject, the pressurization of the wearable cuff is achieved by use of a pump. An example of a system that uses such a pump is described in US 2010/0106029. However, due to the large overpressure and air volume that needs to be generated in order to pressurize the wearable cuff, the pump that is required in existing techniques is particularly large and noisy. The pump that is required in existing techniques also has a high power consumption. Even in the case of the smaller wearable cuff (e.g. for a finger), which intrinsically has only a small cuff-volume, pressurization of the wearable cuff results in high power consumption, since the pump is required to pump against a large difference in pressure between that in the wearable cuff and the environmental (or atmospheric) pressure. Accordingly, a reasonably large and powerful pump is even required in the case of a smaller wearable cuff. Thus, due to the large and powerful pump that is required for pressurization, existing systems for pressurizing a wearable cuff are generally obtrusive and bulky, which means that the existing systems cannot be realized in wearable form.

SUMMARY OF THE INVENTION

As noted above, a limitation with existing systems is that they require large and powerful pumps for pressurization of the wearable cuff in measuring a physiological characteristic of a subject. The pumps can thus be noisy and result in the systems generally being obtrusive and bulky. It would thus be valuable to have an improvement to address the existing problems.

Therefore, according to a first aspect, there is provided a system for use with a wearable cuff in measuring a physiological characteristic of a subject. The system comprises a chamber configured to hold a fluid at a pressure that is greater than atmospheric pressure and a valve and/or a micro pump configured to couple the chamber to the cuff. The valve and/or micro pump is operable to control fluid flow between the chamber and the cuff to increase the pressure in the cuff to a predetermined threshold pressure and vary the pressure in the cuff around the predetermined threshold pressure.

In some embodiments, the valve and/or micro pump may be operable to vary the pressure in the cuff by being operable to increase the pressure in the cuff above the predetermined threshold pressure to inflate the cuff and/or decrease the pressure in the cuff below the predetermined threshold pressure to deflate the cuff. In some embodiments, the predetermined threshold pressure may be a pressure at which the difference between the pressure in the chamber and the pressure in the cuff is less than a threshold.

In some embodiments, the valve and/or micro pump may be operable to vary the pressure in the cuff around the predetermined threshold pressure on the basis of a measured physiological characteristic of the subject. In some embodiments, the valve and/or micro pump may be operable to vary the pressure in the cuff around the predetermined threshold pressure on the basis of oscillations in the measured physiological characteristic of the subject.

In some embodiments, the chamber may be configured to hold a fluid adsorbing material. In some embodiments, the fluid adsorbing material may comprise a zeolite. In some embodiments, a volume of the chamber may be less than or equal to a volume of the cuff. In some embodiments, the system may comprise a further valve connecting the chamber to the atmosphere. In some of these embodiments, the further valve may be configured to allow fluid to enter the chamber to increase the pressure in the chamber above atmospheric pressure.

In some embodiments, the system may comprise the cuff. In some embodiments, the cuff may comprise a first surface configured to contact with the skin of the subject in use and a second surface on which the chamber is positioned, wherein the second surface is opposite the first surface. In some embodiments, the chamber and the cuff may form a closed system in use. In some embodiments, the system may comprise a controller configured to operate the valve and/or micro pump to control the fluid flow between the chamber and the cuff.

According to a second aspect, there is provided a method of operating a system for use with a wearable cuff in measuring a physiological characteristic of a subject. The system comprises a chamber configured to hold a fluid at a pressure that is greater than atmospheric pressure and a valve and/or a micro pump configured to couple the chamber to the cuff and operable to control fluid flow between the chamber and the cuff. The method comprises increasing the pressure in the cuff to a predetermined threshold pressure and varying the pressure in the cuff around the predetermined threshold pressure.

According to a third aspect, there is provided a computer program product comprising a computer readable medium, the computer readable medium having computer readable code embodied therein, the computer readable code being configured such that, on execution by a suitable computer or processor, the computer or processor is caused to perform the method described above.

According to the aspects and embodiments described above, the limitations of existing techniques are addressed. In particular, according to the above-described aspects and embodiments, a chamber that is configured to couple to the wearable cuff holds a fluid at a pressure that is greater than atmospheric pressure and pressurization of the wearable cuff is controlled by the control of fluid flow between the chamber and the cuff. In this way, minimal energy is needed for pressurization of the wearable cuff since the overpressure in the chamber causes the wearable cuff to be filled to a predetermined threshold pressure before the pressure is varied around that predetermined threshold pressure. Moreover, the pressure can also be varied with minimal energy since the pressure difference between the chamber and the wearable cuff is small (or even zero) once the fluid flow from the chamber to the wearable cuff has resulted in the predetermined threshold pressure having been reached.

Therefore, as minimal energy is required, the need for large and powerful pumps is eliminated. Instead, a micro pump (and optionally also a valve) can be used for pressurization of the wearable cuff or a pump can even be eliminated entirely through use of a valve alone for pressurization of the wearable cuff. The system can thus be miniaturized such that it is less obtrusive and can be made more portable (or even wearable). The system also consumes less power and operates with a reduced noise level or even noiselessly due to the elimination or miniaturization of the pump.

There is thus provided an improved system and method for use with a wearable cuff in measuring a physiological characteristic of a subject, which is aimed at overcoming existing problems.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a block diagram of a system according to an embodiment;
Fig. 2 is a block diagram of a system according to another embodiment;
Fig. 3 (a)-(b) illustrate block diagrams of a system according to another embodiment;
Fig. 4 is flow chart illustrating a method according to an embodiment;
Fig. 5 is a graphical illustration of a pressure as a function of a volume ratio according to an embodiment; and
Fig. 6 is a graphical illustration of a pressure as a function of a volume ratio according to another embodiment.

DETAILED DESCRIPTION OF EMBODIMENTS

As noted above, there is provided herein an improved system and method for use with a wearable cuff in measuring a physiological characteristic of a subject, which is aimed at overcoming existing problems. The wearable cuff referred to herein is a cuff that is configured to be worn around a part of a body of a subject. The part of the body of the subject can, for example, be a digit (e.g. a finger, a thumb or a toe) of the subject, an arm (e.g. an upper arm) of the subject, or any other part of the body of the subject. The subject referred to herein can, for example, be a patient, a user of the cuff, or any other subject.

Briefly, the system described herein comprises a chamber (e.g. a reservoir) configured to hold a fluid at a pressure that is greater than (or in excess of) atmospheric pressure. A pressure greater than atmospheric pressure is known in the art as an overpressure. Thus, the chamber referred to herein can be referred to as an overpressure chamber. The overpressure in the chamber is defined as the pressure in the chamber, which is greater than atmospheric pressure. The fluid referred to herein can comprise any fluid that is suitable of being held by the chamber at a pressure that is greater than atmospheric pressure. For example, in some embodiments, the fluid can comprise one or more gases, such as any one or more of normal (or ambient) air, carbon dioxide, nitrogen dioxide, nitrogen, one or more noble gases (e.g. any one or more of helium, neon, argon, krypton and xenon), or any other gas, or any combination of gases. Alternatively or in addition to one more gases, the fluid can comprise one or more liquids, such as water (e.g. enriched with detergent or soap), or any other liquid, or any combination of liquids.

In addition to the chamber configured to hold the fluid, the system described herein also comprises a valve and/or a micro pump configured to couple the chamber to the cuff. Thus, the chamber and cuff are coupled (e.g. connected) via the valve and/or the micro pump. The valve and/or the micro pump is operable to control fluid flow between the chamber and the cuff. In particular, the valve and/or the micro pump is operable to control fluid flow between the chamber and the cuff to increase the pressure in the cuff to a predetermined threshold pressure and vary the pressure in the cuff around the predetermined threshold pressure. In this way, the valve and/or the micro pump can be used to inflate the cuff and further to adapt the pressure in the cuff around the predetermined threshold pressure. The inflation of the cuff around a part of the body of a subject compresses the enclosed tissue of that part of the body. This compression of the enclosed tissue can allow for measurement of one or more physiological characteristics (e.g. a blood pressure, or any other physiological characteristic, or any combination of physiological characteristics) of a subject.

In some embodiments, the predetermined threshold pressure referred to herein may be a pressure at which the difference between the pressure in the chamber and the pressure in the cuff is less than a threshold or zero (or approximately zero). For example, in some embodiments, the predetermined threshold pressure may be a pressure at which an equilibrium is reached, where the pressure in the cuff is equal to or approximately equal to the pressure in the chamber. As mentioned earlier, the predetermined threshold pressure may also be referred to as a minimum (or static) pressure. In some embodiments, the predetermined threshold pressure may be a value that corresponds to the maximum (or peak) of the pressure oscillations in the wearable cuff, induced by the heart beat of the subject.

The micro pump described herein that is operable to control fluid flow between the chamber and the cuff can be operable to control the fluid flow by pumping the fluid between the chamber and the cuff, e.g. from the chamber to the cuff (such as to inflate and pressurize the cuff) and/or from the cuff to the chamber (such as to depressurize the cuff). The micro pump may be located anywhere between the chamber and the cuff in the system, such as at an outlet of the chamber, in a component configured to couple the chamber to the cuff, or at any other location between the chamber and the cuff. A person skilled in the art will be aware of various micro pumps that may be used. Examples of a micro pump that may be used include, but are not limited to, a micro iron-core motor pump, a micro eccentric diaphragm pump, a micro gas pump, a micro piezoelectric diaphragm pump, a micro membrane pump, or any other micro pump, or any combination of micro pumps.

The valve described herein that is operable to control fluid flow between the chamber and the cuff may be referred to as a controllable flow valve. In some embodiments, the valve referred to herein that is operable to control fluid flow between the chamber and the cuff can be operable to switch between an open position (where fluid can flow between the chamber and the cuff) and a closed position (where fluid is prevented from flowing between the chamber and the cuff). This type of valve may be referred to as a switchable open/close valve.

In embodiments where the system comprises a valve operable to control fluid flow between the chamber and the cuff, the valve operable to control fluid flow between the chamber and the cuff may allow the predetermined threshold pressure to be reached more quickly. Thus, the valve operable to control fluid flow between the chamber and the cuff can allow the cuff to be inflated faster (e.g. the valve can increase the velocity at which the cuff is inflated) according to some embodiments.

In some embodiments where the system comprises a valve and a micro pump operable to control fluid flow between the chamber and the cuff, the valve operable to control fluid flow between the chamber and the cuff may be operable to fully decouple or at least reduce the (over)pressure that the micro pump is exposed to before inflation of the cuff. In some embodiments where the system comprises a valve and a micro pump operable to control fluid flow between the chamber and the cuff, the valve operable to control fluid flow between the chamber and the cuff may be positioned in series with or parallel to the pump operable to control fluid flow between the chamber and the cuff.

In some embodiments where the system comprises a valve operable to control fluid flow between the chamber and the cuff without a pump, the valve may be operable to control fluid flow from the chamber to the cuff (e.g. to inflate and pressurize the cuff) and from the cuff to the chamber (e.g. to depressurize the cuff). In some of these embodiments, a first valve may be operable to control fluid flow from the chamber to the cuff (e.g. to inflate and pressurize the cuff) and a second valve may be operable to control fluid flow from the cuff to the chamber (e.g. to depressurize the cuff). The cycle of fluid flow from the chamber to the cuff and vice versa may be restarted, if required. It is possible to realize a system without a pump and with only one or more controllable valves according to some embodiments since the pressure oscillations induced in the wearable cuff by the heart beat of the subject are only a few mbar. In some embodiments, the overpressure in the chamber may be higher in order to compensate for pressure-dissipation during the oscillation cycles. A system without a pump can generate no noise and has minimum power consumption.

In some embodiments, the overpressure in the chamber may be higher than that required to generate a pressure that corresponds to a minimum in the pressure oscillations in the wearable cuff, induced by the heart beat of the subject. For example, in some embodiments, the overpressure in the chamber may be greater than 4.391 bar. For example, in some embodiments, the overpressure in the chamber may be sufficiently high that the maximum pressure during the oscillations can be reached without using a pump. Accordingly, in some embodiments, a valve may be used to allow fluid to flow from the chamber to the cuff and a micro pump may only be used to pump back fluid from the cuff to the chamber in order to control the fluid flow in the manner described herein.

Although the system is described herein as comprising "a micro pump" and/or "a valve" operable to control fluid flow between the chamber and the cuff, it will be understood that the system may comprise one or more micro pumps (i.e. a single micro pump or a plurality of micro pumps) and/or one or more valves (i.e. a single valve or a plurality of valves) operable to control fluid flow between the chamber and the cuff. Similarly, although the system is described as comprising "a chamber" configured to hold a fluid at a pressure that is greater than atmospheric pressure, it will be understood that the system may comprise one or more chambers (i.e. a single chamber or a plurality of chambers) configured to hold a fluid at a pressure that is greater than atmospheric pressure.

In some embodiments where the system comprises a plurality of chambers configured to hold a fluid at a pressure that is greater than atmospheric pressure, the plurality of chambers may hold fluid at the same pressure (which is greater than atmospheric pressure) or at different pressures (which are each greater than atmospheric pressure). In some of these embodiments, one or more chambers may hold a fluid, which is controlled to flow between the chamber and the cuff. In other embodiments, at least one chamber may be configured to hold a fluid, which is only controlled to flow between the chamber and the cuff if compensation is needed, e.g. where the fluid held in one or more other chambers is insufficient.

In any of the embodiments described herein, the system 100 can further comprise the cuff 200. Alternatively or in addition, in any of the embodiments described herein, the chamber 102 and the cuff 200 can form a closed system in use. That is, in some embodiments, the system 100 may be a closed system or, more specifically, a closed pressurizing system.

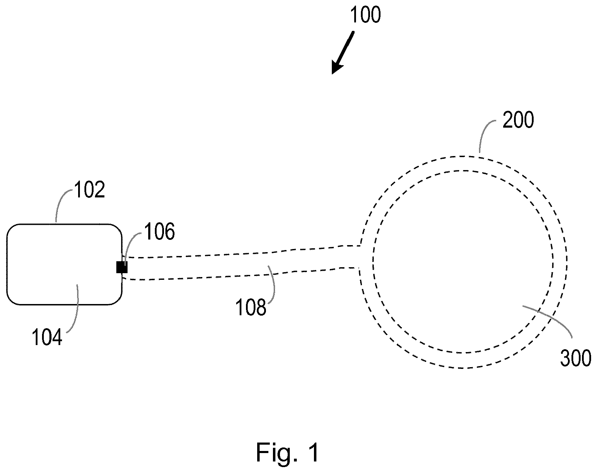

Fig. 1 illustrates a system 100 for use with a wearable cuff 200 according to an embodiment. More specifically, Fig. 1 illustrates a cross-sectional view of the system 100 around a part of a body of a subject 300. The system 100 is for use with the cuff 200 in measuring a physiological characteristic of the subject 300. As illustrated in Fig. 1, the system 100 comprises a chamber 102. The chamber 102 is configured to hold a fluid 104 at a pressure that is greater than atmospheric pressure. As also illustrated in Fig. 1, the system 100 further comprises a valve and/or a micro pump 106.

The valve and/or the micro pump 106 is configured to couple the chamber 102 to the cuff 200. Thus, the chamber 102 and cuff 200 are coupled (e.g. connected) via the valve and/or the micro pump 106.

The valve and/or the micro pump 106 is operable to control fluid flow between the chamber 102 and the cuff 200. In particular, the valve and/or the micro pump 106 is operable to control fluid flow between the chamber 102 and the cuff 200 to increase the pressure in the cuff 200 to a predetermined threshold pressure and vary the pressure in the cuff 200 around the predetermined threshold pressure.

As illustrated in Fig. 1, according some embodiments, the valve and/or the micro pump 106 can be configured to couple the chamber 102 to the cuff 200 via one or more other components 108. Thus, in some embodiments, the chamber 102 and the cuff 200 can be coupled (e.g. connected) via the valve and/or micro pump 106 and one or more other components 108. The one or more other components 108 can comprise any component suitable for allowing fluid to flow through it. For example, the one or more other components 108 can comprise a tube, or any other component, or any combination of components. In other embodiments, the valve and/or the micro pump 106 can be configured to couple the chamber 102 to the cuff 200 directly. Thus, the chamber 102 and the cuff 200 can be coupled (e.g. connected) via the valve and/or micro pump 106 alone according to some embodiments.

The system 100 illustrated in Fig. 1 will now be described by way of a mathematical example. In this example, before pressurization of the cuff 200, it is assumed that there are normal atmospheric conditions in the cuff 200. More specifically, for the purpose of this example, it is assumed that the starting (or normal) pressure $P_2$ in the cuff 200 is 1 bar, while the volume $V_2$ of the cuff 200 is 1 dm$^3$. It is also assumed that the volume $V_0$ of the cuff 200 will increase by 20% of its original volume $V_2$ while a static pressure of approximately 80 mmHg (which is a typical diastolic blood pressure) is applied to the cuff 200. That is, $V_0 = 1.2 \cdot V_2 = 1.2$ dm$^3$. In addition, the required static pressure (i.e. the predetermined threshold pressure) $P_0$ in the cuff 200 and the whole (closed) system 100 is assumed to be at least 80 mmHg (which corresponds to 80 mmHg / 750 mmHg/bar = 0.107 bar) above the starting pressure $P_2$. That is, $P_0 = P_2$ + 80 mmHg = 1 bar + 80 mmHg = 1 bar + 0.107 bar = 1.107 bar. The volume $V_1$ of the chamber 102 is assumed to be 10% of the volume $V_2$ of the cuff 200. That is, $V_1 = 0.1 \cdot V_2 = 0.1 \cdot 0.1$ dm$^3$ = 0.01 dm$^3$.

With this information, the required overpressure $P_1$ in the chamber 102 can be estimated. That is, the pressure $P_1$ at which the chamber 102 is configured to hold the fluid 104, which is greater than atmospheric pressure, can be estimated. Basis for this estimation can be provided by the (ideal) gas-law of Boyle-Mariotte:

$$P \cdot V = constant.$$

Before pressurizing the cuff 200, the volume $V_1$ of the chamber 102 as well as the starting pressure $P_2$ in the cuff 200 with volume $V_2$ is given. If the cuff 200 is inflated to a predetermined threshold pressure (which in this mathematical example is the minimum pressure), the whole (closed) system 100 has approximately the same pressure Po, whereby the whole volume of the system 100 consists of the volume $V_1$ and the slightly expanded cuff volume $V_0$. The volume of the other components (e.g. a tube) 108 are ignored to simplify the mathematical example. Thus, applying the (ideal) gas-law from Boyle-Mariotte to the (closed) system 100 according to this mathematical example:

$$P_1 \cdot V_1 + P_2 \cdot V_2 = P_0 \cdot (V_1 + V_0),$$

where the left side of the equation is before inflation of the cuff 200 and the right side of the equation is after inflation of the cuff 200. According to this mathematical example, this gives:

$$P_1 \cdot 0.1 \cdot V_2 + P_2 \cdot V_2 = P_0 \cdot (0.1 \cdot V_2 + 1.2 \cdot V_2).$$

This can be simplified to provide the required overpressure $P_1$ in the chamber 102 according to this mathematical example as:

$$P_1 = 10 \cdot (1.3 \cdot P_0 - P_2).$$

By substituting the assumed values into this equation, the required overpressure $P_1$ in the chamber 102 is determined to be 10·(1.439 - 1) bar = 4.391 bar. From the equations above, it can be derived that, by increasing the volume of the chamber 102, the required overpressure is halved. This shows that only a reasonable overpressure in a small chamber 102 is required to pressurize the cuff 200. In addition to the small volume, in some embodiments, a higher freedom of design to realize and localize the chamber 102 in the vicinity of the cuff 200 may be possible. For example, in some embodiments, the chamber 102 can be shaped (e.g. bent) to fit around the cuff 200.

Since much higher pressures can be stored in the chamber 102 described herein, the whole pre-pressurizing cycle (where the pressure in the cuff 200 is increased to the predetermined threshold pressure) may be repeated after a predetermined time or the pressure in the cuff 200 may be corrected. For example, in some embodiments, the pressure in the cuff 200 may be increased if the blood pressure of the subject 300 starts to increase. Similarly, in some embodiments, a decrease in the blood pressure of the subject 300 may be compensated by reducing the pressure in the cuff 200.

Continuing the mathematical example, an additional increase in the pressure in the cuff 200 of $P_0' = 6.667$ mbar resulting in an additional increase of the volume of the cuff 200 of 2% ($\Delta V_0 = 0.02 \cdot V_0$) is assumed. With $P_0$ equal to the predetermined threshold pressure after the increase in pressure in the cuff 200, a resulting peak-vacuum in the chamber 102 can be calculated, as follows:

$$P_0 \cdot (V_1 + V_0) = P_1' \cdot V_1 + (P_0 + P_0') \cdot (V_0 + V_0').$$

The resulting peak-vacuum is the minimum (or lowest) pressure $P'_1$ occuring in the chamber 102 as a result of the increase in pressure in the cuff 200 to the predetermined threshold pressure. The resulting peak-vacuum is a pressure $P'_1$ that is less than the initial overpressure $P_1$ in the chamber 102. In some embodiments, the resulting peak-vacuum may still be greater than atmospheric pressure.

From this, the minimum pressure $P'_1$ to be generated in the chamber 102 while the pressure in the cuff 200 is varied (e.g. oscillated) around the predetermined threshold pressure can be determined, as follows:

$$P_1' = \frac{1}{0.1 \cdot V_2} \cdot [P_0 \cdot (0.1V_2 + 1.2V_2) - (P_0 + P_0') \cdot (1.2V_2 + 0.02 \cdot 1.2V_2)].$$

This gives the minimum pressure $P'_1$ as:

$$P_1' = \frac{1}{0.1} \cdot [P_0 \cdot (1.3) - (P_0 + P_0') \cdot (1.224)] = 10 \cdot (0.076 \cdot P_0 - 1.224 \cdot P_0').$$

Taking the equilibrium pressure Po= 1.107 bar and the maximum pressure increase in the cuff $P_0'$ = 6.667 mbar, the pressure in the chamber 102 can be calculated to be 0.76 bar. Thus, a vacuum (with a pressure $P'_1$ below the initial overpressure) is being generated in the chamber 102.

The system 100 illustrated in Fig. 1 will now be described by way of another mathematical example. In this example, before pressurization of the cuff 200, it is assumed that there are normal atmospheric conditions in the cuff 200. More specifically, for the purpose of this example, it is assumed that the starting (or normal) pressure $P_2$ in the cuff 200 is 1 bar, while the volume $V_2$ of the cuff 200 is 1 cm$^3$ (which is much smaller than the volume $V_2$ of the cuff 200 in the earlier mathematical example). It is also assumed that the volume $V_0$ of the cuff 200 will increase by 10% of its original volume $V_2$ while a static pressure of approximately 80 mmHg (which is a typical diastolic blood pressure) is applied to the cuff 200, which is a much lower increase than in the earlier mathematical example. That is, in this mathematical example, $V_0 = 1.1 \cdot V_2 = 1.1$ cm$^3$. In addition, the required static pressure (i.e. the predetermined threshold pressure) $P_0$ in the cuff 200 and the whole (closed) system 100 is assumed to be at least 80 mmHg (which corresponds to 80 mmHg / 750 mmHg/bar = 0.107 bar) above the starting pressure $P_2$. That is, $P_0 = P_2 + 80$ mmHg = 1 bar + 80 mmHg = 1 bar + 0.107 bar = 1.107 bar. The volume $V_1$ of the chamber 102 is parameterized to be 10% of the volume $V_2$ of the cuff 200. Further, the volume of the chamber 102 is parameterized with a variable "x". That is, $V_1 = x \cdot V_2$.

With this information, the required overpressure $P_1$ in the chamber 102 can be estimated. That is, the pressure $P_1$ at which the chamber 102 is configured to hold the fluid 104, which is greater than atmospheric pressure, can be estimated. Thus, as in the earlier mathematical example, applying the (ideal) gas-law from Boyle-Mariotte to the (closed) system 100 according to this mathematical example:

$$P_1 \cdot V_1 + P_2 \cdot V_2 = P_0 \cdot (V_1 + V_0),$$

where the left side of the equation is before inflation of the cuff 200 and the right side of the equation is after inflation of the cuff 200. According to this mathematical example, this gives:

$$P_1 \cdot x \cdot V_2 + P_2 \cdot V_2 = P_0 \cdot (x \cdot V_2 + 1.1 \cdot V_2).$$

This can be simplified to provide the required overpressure $P_1$ in the chamber 102 according to this mathematical example as:

$$P_1 = \frac{1}{x} \cdot [P_0 \cdot (1.1 + x) - P_2].$$

By setting x to 0.5 (such that the volume of the chamber 102 is half the volume of the cuff 200), the required overpressure $P_1$ in the chamber 102 is 1.54 bar. Increasing the volume of the chamber 102, such that it is equal to the volume of the cuff 200 (i.e. setting x to 1), the required overpressure $P_1$ in the chamber 102 is 1.32 bar. This mathematical example shows that the (closed) system 100 can be realized with reasonable or small overpressure in the chamber 102.

Continuing the example, an additional increase in the pressure in the cuff 200 of $P_0' = 6.667$ mbar resulting in an additional increase of the volume of the cuff 200 of 1% ($\Delta V_0 = 0.01 \cdot V_0$) is assumed. With $P_0$ equal to the predetermined threshold pressure after the increase in pressure in the cuff 200, a resulting peak-vacuum in the chamber 102 can be calculated, as follows:

$$P_0 \cdot (V_1 + V_0) = P_1' \cdot V_1 + (P_0 + P_0') \cdot (V_0 + V_0').$$

As mentioned in the previous example, the resulting peak-vacuum is the minimum (or lowest) pressure $P_1'$ occuring in the chamber 102 as a result of the increase in pressure in the cuff 200 to the predetermined threshold pressure. The resulting peak-vacuum is a pressure $P_1'$ that is less than the initial overpressure $P_1$ in the chamber 102. In some embodiments, the resulting peak-vacuum may still be greater than atmospheric pressure.

From this, a minimum pressure $P_1'$ to be generated in the chamber 102 while the pressure in the cuff 200 is varied (e.g. oscillated) around the predetermined threshold pressure can be determined, as follows:

$$P_1' = \frac{1}{x \cdot V_2} \cdot [P_0 \cdot (x \cdot V_2 + 1.1 V_2) - (P_0 + P_0') \cdot (1.1 V_2 + 0.01 \cdot 1.1 V_2)].$$

This gives the minimum pressure $P_1'$ as:

$$P_1' = \frac{1}{x} \cdot [P_0 \cdot (x + 1.1) - (P_0 + P_0') \cdot (1.111)] = \frac{1}{x} \cdot [P_0(x - 0.011) - 1.111 \cdot P_0'].$$

Taking the equilibrium pressure Po= 1.107 bar and the maximum pressure increase in the cuff $P_0' = 6.667$ mbar, the remaining pressure in the chamber 102 can be calculated. As before, two values for x are considered. For x= 0.5 (where the volume of the chamber 102 is half the volume of the cuff 200), the required pressure is 1.068 bar. For x= 1 (where the volume of the chamber 102 is equal to the volume of the cuff 200), the required pressure is 1.087 bar. This mathematical example shows that no vacuum needs to be realized as the pressure in the chamber 102 is still greater than atmospheric pressure.

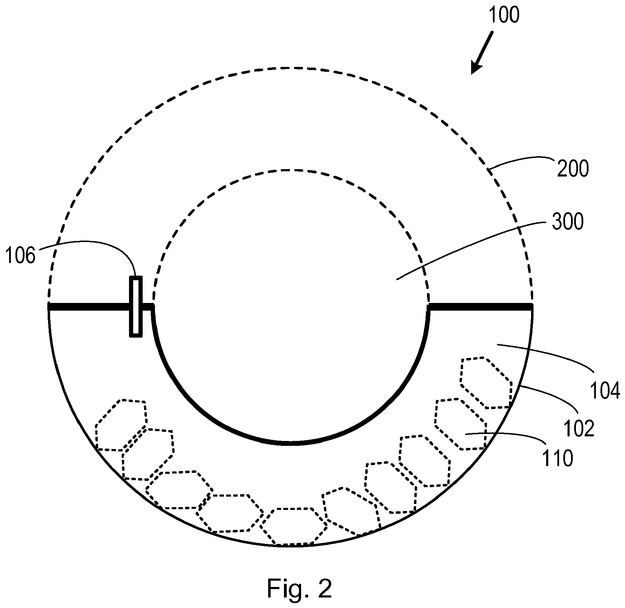

Fig. 2 illustrates a system 100 for use with a wearable cuff 200 according to another embodiment. More specifically, Fig. 2 illustrates a cross-sectional view of the system 100 around a part of the body of a subject 300. The system 100 is for use with the cuff 200 in measuring a physiological characteristic of the subject 300. As illustrated in Fig. 2, the system 100 comprises a chamber 102. The chamber 102 is configured to hold a fluid 104 at a pressure that is greater than atmospheric pressure. As also illustrated in Fig. 2, the system 100 further comprises a valve and/or a micro pump 106. The valve and/or the micro pump 106 is configured to couple the chamber 102 to the cuff 200. Thus, the chamber 102 and cuff 200 are coupled (e.g. connected) via the valve and/or the micro pump 106.

The valve and/or the micro pump 106 is operable to control fluid flow between the chamber 102 and the cuff 200. In particular, the valve and/or the micro pump 106 is operable to control fluid flow between the chamber 102 and the cuff 200 to increase the pressure in the cuff 200 to a predetermined threshold pressure and vary the pressure in the cuff 200 around the predetermined threshold pressure.

As illustrated in Fig. 2, according some embodiments, the valve and/or the micro pump 106 can be configured to couple the chamber 102 to the cuff 200 directly. Thus, the chamber 102 and the cuff 200 can be coupled (e.g. connected) via the valve and/or micro pump 106 alone according to some embodiments. In other embodiments, the valve and/or the micro pump 106 can be configured to couple the chamber 102 to the cuff 200 via one or more other components (as described earlier with reference to Fig. 1).

As also illustrated in Fig. 2, in some embodiments, the chamber 102 can be configured to hold a fluid adsorbing material 110. This adsorbing material may be selected such that it will not only adsorb fluids in case of a (partial) pressure increase in the chamber 102, but can also desorb the fluid in case of a (partial) pressure decrease in the chamber 102. In embodiments where the fluid comprises one or more gases, the presence of the adsorbing material 110 can provide an effective capacity increase of the chamber 102 without increasing the dimensions of the chamber 102 (e.g. in terms of the volume or size of the chamber 102). The fluid adsorbing material 110 can be any material suitable for adsorbing the fluid 104. The fluid adsorbing material 110 increases the effective volume of the chamber 102. For example, in some embodiments, the fluid adsorbing material 110 can increase the effective volume of the chamber 102 by more than a certain factor (e.g. more than a factor of 3). In some embodiments, the fluid adsorbing material 110 may comprise a molecular sieve. For example, according to some embodiments, the fluid adsorbing material 110 may comprise a zeolite (such as any of those used in a gas concentrator), or any other fluid adsorbing material, or any combination of fluid adsorbing materials.

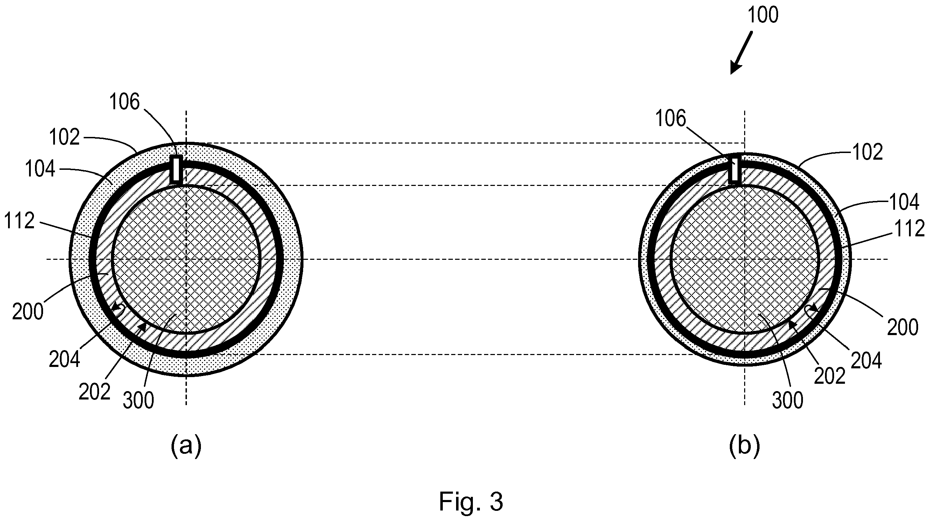

Fig. 3 illustrates a system 100 for use with a wearable cuff 200 according to another embodiment. More specifically, Fig. 3 illustrates a cross-sectional view of the system 100 around a part of the body of a subject 300. The system 100 is for use with the cuff 200 in measuring a physiological characteristic of the subject 300. As illustrated in Fig. 3, the system 100 comprises a chamber 102. In this embodiment, the chamber 102 is adapted to be positioned around the cuff 200. That is, the chamber 102 can form a shell-like configuration around the cuff 200. As illustrated in Fig. 3, in some embodiments, the chamber 102 can be adapted to be positioned around an entire circumference of the cuff 200. However, it will be appreciated that, in other embodiments, the chamber 102 may be adapted to be positioned around only a part of a circumference of the cuff 200. The chamber 102 is configured to hold a fluid 104 at a pressure that is greater than atmospheric pressure.

As also illustrated in Fig. 3, the system 100 further comprises a valve and/or a micro pump 106. The valve and/or the micro pump 106 is configured to couple the chamber 102 to the cuff 200. Thus, the chamber 102 and cuff 200 are coupled (e.g. connected) via the valve and/or the micro pump 106. The valve and/or the micro pump 106 is operable to control fluid flow between the chamber 102 and the cuff 200. In particular, the valve and/or the micro pump 106 is operable to control fluid flow between the chamber 102 and the cuff 200 to increase the pressure in the cuff 200 to a predetermined threshold pressure and vary the pressure in the cuff 200 around the predetermined threshold pressure.

Fig. 3(a) illustrates the system 100 in a normal pressurized state, where the fluid 104 is held in the chamber 102 at the pressure that is greater than atmospheric pressure. Fig. 3(b) illustrates the system 100 following pressurization of the cuff 200, where the pressure in the cuff 200 is increased to the predetermined threshold pressure by the valve and/or micro pump 106 controlling the fluid 104 to flow from the chamber 102 to the cuff 200. The changes in size of the system 100 due to the pressurization of the cuff 200 are illustrated by way of the dashed lines in Fig. 3. Although not illustrated in Fig. 3, it will be appreciated that there may be a minimal change in the volume of the cuff 200 due to the pressurization of the cuff 200.

As illustrated in Fig. 3, according some embodiments, the valve and/or the micro pump 106 can be configured to couple the chamber 102 to the cuff 200 directly. Thus, the chamber 102 and the cuff 200 can be coupled (e.g. connected) via the valve and/or micro pump 106 alone according to some embodiments. In other embodiments, the valve and/or the micro pump 106 can be configured to couple the chamber 102 to the cuff 200 via one or more other components (as described earlier with reference to Fig. 1).

As also illustrated in Fig. 3, in some embodiments, the cuff 200 may comprise a first surface 202 configured to contact with the skin of the subject 300 in use and a second surface 204 on which the chamber 102 is positioned. In these embodiments, as illustrated in Fig. 3, the second surface 204 is opposite the first surface 202. As also illustrated in Fig. 3, in some embodiments the chamber 102 and the cuff 200 may be separated by a first material 112. The first material 112 separating the chamber 102 and the cuff 200 may be a rigid (or stiff) material. In use, the first material 112 may stay a constant or relatively constant size. Alternatively or in addition to the first material 112, in some embodiments, the first surface 202 of the cuff 200 may be formed from or covered by a second material (not illustrated in Fig. 3). Thus, in some embodiments, the cuff 200 and the part of the body of the subject 300 around which the cuff 200 is worn may be separated by the second material in use. The second material may be a flexible material. Thus, in embodiments having both the first material 112 and the second material, the rigidity of the first material 112 can be greater than the rigidity of the second material.

Alternatively or in addition to the first material 112 and/or the second material, in some embodiments, the outer surface of the chamber 102 may be formed from or covered by a third material (not illustrated in Fig. 3). Thus, in some embodiments, the chamber 102 and the atmosphere may be separated by the third material. The third material may be a flexible material. Thus, in embodiments having both the first material 112 and the third material, the rigidity of the first material 112 can be greater than the rigidity of the third material. The use of a flexible third material allows the volume of the chamber 102 to reduce. This can be advantageous, for example, if a lower pressure (e.g. a vacuum) needs to be generated in the chamber 102, such as when the fluid flow between the chamber 102 and the cuff 200 is controlled to vary the pressure in the cuff 200 around the predetermined threshold pressure. In this way, in embodiments where the system 100 comprises a micro pump 106, an even smaller micro pump can be used.

Although the embodiments illustrated in Figs. 1, 2 and 3 have each been described separately, it will be understood that any of the features of one of those embodiments, may also be present in one or more of the other embodiments. Moreover, it will be appreciated that Figs. 1, 2 and 3 only show the components required to illustrate some embodiments and, in a practical implementation, the system 100 may comprise additional or alternative components to those shown.

In any embodiment (such as any of those described earlier with reference to Figs. 1, 2 and 3), a volume of the chamber 102 may be less than or equal to a volume of the cuff 200. In this way, the system 100 can be compact in size. In some embodiments, the volume of the chamber 102 may differ from the volume of the cuff 200 by less than a threshold. For example, the volume of the chamber 102 may be close to the volume of the cuff 200 according to some embodiments. In embodiments where the system 100 comprises a pump, the efficiency of the pump can be improved in this way.

In an embodiment where the system 100 comprises a pump, for a given volume in the cuff 200 to be inflated, the power consumption of the pump can be correlated with the pressure ratio between a starting pressure p0 in the chamber 102 and an end pressure p1 in the cuff 200. Assuming the operation of the pump is 100% efficient, the power consumption of (or work to be performed by) the pump $P_{pump}$ is proportional to:

$$P_{pump} \sim \Phi * [\ (p_1/p_0)^{((\kappa-1/\kappa))} - 1\ ],$$

where $\kappa = C_p/C_v$, C is the specific heat capacity of the fluid held in the chamber 102, and $\Phi_{flluid}$ is the fluid flow. Working close to atmospheric pressure (in atm) and using a fluid flow $\Phi_{fluid}$ given in units L/min, the power consumption of (or work to be performed by) the pump $P_{pump}$ [in W] can be approximated by:

$$P_{pump} = 1.58 * \Phi_{fluid} * (p_1 - p_0).$$

The pump inflow needed to pressurize a cuff volume of 10 mL from 1.1 atm to 1.11 atm is 0.1 mL. This increase in pressure may be performed in about 3 s, and 3 s may be used to deflate the cuff 200 again (e.g. without using the pump). Therefore, the required fluid flow $\Phi_{fluid}$ is 1 mL/min. When the pump is running against air pressure, the power $P_{pump}$ required for the pump to deliver a fluid flow of 1 mL/min is 0.17 mW. In comparison, when the pump is running against the volume of the chamber 102, the power $P_{pump}$ required for the pump to deliver a fluid flow of 1 mL/min is 0.032 mW. This assumes that the volume of the chamber 102 is same as the volume of the cuff 200 and the volume of the chamber 102 is maintained at a lowest pressure $p_0$ of 1.09 atm. There is thus a significant decrease in power consumption achieved by using the chamber 102 described herein.

In any embodiment (such as any of those described earlier with reference to Figs. 1, 2 and 3), the chamber 102 may be a replaceable (and optionally also a disposable) chamber, e.g. a replaceable (and optionally also a disposable) cartridge. That is, the chamber 102 may be configured to be removed from the system 100 and replaced with a replacement chamber 102. In these embodiments, the replacement chamber 102 is also configured to hold a fluid at a pressure that is greater than atmospheric pressure.

In any embodiment (such as any of those described earlier with reference to Figs. 1, 2 and 3), although not illustrated, the system 100 may comprise a further valve connecting the chamber 102 to the atmosphere or an external fluid supply. In these embodiments, the further valve can be configured to allow fluid 104 to be input into the chamber 102 to increase the pressure in the chamber 102 above atmospheric pressure. Alternatively or in addition, the further valve can be configured to allow fluid 104 to be input into the chamber 102 to increase the pressure in the chamber 102 and aid in increasing the pressure in the cuff 200 to the predetermined threshold pressure. Thus, in some embodiments, the atmosphere or an external fluid supply can act as a supporting volume for pressurizing the cuff 200. This can be useful, for example, where the effective volume of the chamber 102 is small.

Alternatively or in addition, the further valve can be configured to allow fluid 104 to be released (or exhausted) from the chamber 102 to reduce the pressure in the chamber 102. For example, the further valve can be configured to open to the release (or exhaust) the fluid 104 from the chamber 102. The fluid 104 may, for example, be released into the atmosphere or into another chamber. In embodiments where the further valve is configured to allow fluid 104 to be input into the chamber 102, the further valve may also be configured to later release a corresponding amount (e.g. the same amount) of fluid 104, such as during deflation of the cuff 200. In some embodiments, the further valve may be configured to release the fluid 104 from the chamber 102 to reduce the pressure in the chamber 102 down to a value that corresponds to the minimum of the pressure oscillations induced in the wearable cuff 200 by the heart beat of the subject 300.

In any embodiment (such as any of those described earlier with reference to Figs. 1, 2 and 3), although not illustrated, the system 100 can comprise a controller configured to operate the valve and/or micro pump 106 to control the fluid flow between the chamber 102 and the cuff 200 in the manner described herein.

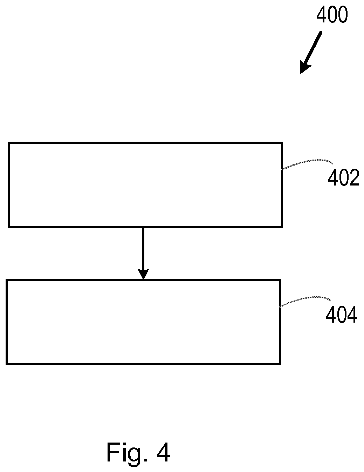

Fig. 4 illustrates a method 400 of operating a system 100 (such as any of the embodiments described earlier with reference to Figs. 1, 2 and 3) for use with a wearable cuff 200 in measuring a physiological characteristic of a subject 300. In embodiments where the system 100 comprises a controller, the method 400 may be performed by or under the control of the controller. As described earlier, the system 100 comprises a chamber 102 configured to hold a fluid 104 at a pressure that is greater than atmospheric pressure. As also described earlier, the system 100 further comprises a valve and/or a micro pump 106 configured to couple the chamber 102 to the cuff 200 and operable to control fluid flow between the chamber 102 and the cuff 200.

With reference to Fig. 4, at block 402 of Fig. 4, the pressure in the cuff 200 is increased to a predetermined threshold pressure. More specifically, the valve and/or a micro pump 106 controls fluid flow between the chamber 102 and the cuff 200 to increase the pressure in the cuff 200 increased to the predetermined threshold pressure.

In some embodiments, the predetermined threshold pressure may be a pressure at which the difference between the pressure in the chamber 102 and the pressure in the cuff 200 is less than a threshold or zero (or approximately zero). For example, in some embodiments, the predetermined threshold pressure may be a pressure at which an equilibrium is reached, where the pressure in the cuff 200 is equal to or approximately equal to the pressure in the chamber 102. As mentioned earlier, the predetermined threshold pressure may also be referred to as a minimum (or static) pressure. In some embodiments, the predetermined threshold pressure may be a value that corresponds to the maximum (or peak) of the pressure oscillations in the wearable cuff 200, induced by the heart beat of the subject 300.

At block 404 of Fig. 4, the pressure in the cuff is varied around the predetermined threshold pressure. More specifically, the valve and/or a micro pump 106 controls fluid flow between the chamber 102 and the cuff 200 to vary the pressure in the cuff around the predetermined threshold pressure. In some embodiments, the valve and/or micro pump 106 can be operable to vary the pressure in the cuff 200 by being operable to increase the pressure in the cuff 200 above the predetermined threshold pressure to inflate the cuff 200. Alternatively or in addition, in some embodiments, the valve and/or micro pump 106 can be operable to vary the pressure in the cuff 200 by being operable to decrease the pressure in the cuff 200 below the predetermined threshold pressure to deflate the cuff 200.

In some embodiments, the valve and/or micro pump 106 may be operable to vary the pressure in the cuff 200 around the predetermined threshold pressure on the basis of a measured physiological characteristic (or vital sign) of the subject 300. In embodiments where the system 100 comprises a controller, the controller can be configured to operate the valve and/or micro pump 106 to vary the pressure in the cuff 200 around the predetermined threshold pressure on the basis of a measured physiological characteristic (or vital sign) of the subject 300. The controller may be configured to acquire the measured physiological characteristic of the subject 300 from a physiological characteristic sensor.

In some embodiments, the valve and/or micro pump 106 may be operable to vary the pressure in the cuff 200 around the predetermined threshold pressure on the basis of oscillations in the measured physiological characteristic of the subject 300. The measured physiological characteristic of the subject 300 can comprise any one or more of a blood pressure of the subject 300, a breathing rate (or respiration rate) of the subject 300, or any other measured physiological characteristic of the subject 300, or any combination thereof. Thus, for example, the valve and/or micro pump 106 may be operable to vary the pressure in the cuff 200 around the predetermined threshold pressure on the basis of oscillations caused by the beating of the heart of the subject, the breathing of the subject, or similar.

Below a software code is provided, such that an example of the system 100 described herein can be illustrated. The parameters used in the software code are defined as follows:

"$V_1$":     volume of the chamber 102;

"$V_2$"     volume of the cuff 200;

"$\Delta V_{21}$":     volume increase of the cuff 200 during pressurizing (at equilibrium, where the pressure in the cuff 200 is equal to or approximately equal to the pressure in the chamber 102);

"$\Delta V_{22}$":     volume increase of the cuff 200 during a peak oscillation (induced in the wearable cuff 200 by the heart beat of the subject 300);

"$P_1$":     pressure in the chamber 102 (which is an unknown and to be calculated)

"$P_2$":     starting pressure in the cuff 200 (which may, for example, be a nominal pressure, an ambient pressure, or atmospheric pressure);

"$\Delta P_{21}$":     pressure increase in the cuff 200 during static operation (at equilibrium, where the pressure in the cuff 200 is equal to or approximately equal to the pressure in the chamber 102); and

"$\Delta P_{22}$":   pressure increase in the cuff 200 during a peak oscillation (induced in the wearable cuff 200 by the heart beat of the subject 300). Also, as an input, the following example values are defined:

"$V_2$":   the absolute value (which can be neglected as the results are independent of it)

"$P_2$":   1 bar

"$\Delta P_{21}$":   0.107 bar (which corresponds to a diastolic blood pressure value of 80 mmHg)

Further, the following parameters that describe relationships within the system 100 and that are to be determined are provided:

"a":   size of the chamber 102 or, more specifically, a ratio of the volume $V_1$ of the chamber 102 to the volume $V_2$ of the cuff 200 (a= $V_1/V_2$);

"b":   change (e.g. expansion or increase) in the volume of the cuff 200 due to pressurization during equilibrium (where the pressure in the cuff 200 is equal to or approximately equal to the pressure in the chamber 102); and

"c":   change (e.g. expansion or increase) in the volume of the cuff 200 during a peak oscillation (induced in the wearable cuff 200 by the heart beat of the subject 300).

In the example, equations are determined to calculate the following:

"$P_1$":   a required overpressure in the chamber 102; and

"$P_{12}$":   a resulting vacuum or pressure at a peak oscillation (induced in the wearable cuff 200 by the heart beat of the subject 300).

In the example, the system 100 is assumed to be a closed system. Also, an ideal closed system is assumed and "dead" volumes (e.g. tubes, hoses, etc) are neglected. The following equations are determined for the state of the system 100 in equilibrium (where the pressure in the cuff 200 is equal to or approximately equal to the pressure in the chamber 102) and the state of the system 100 at a peak oscillation (induced in the wearable cuff 200 by the heart beat of the subject 300):

Equilibrium

$$P_1 \cdot V_1 + P_2 \cdot V_2 = (P_2 + \Delta P_{21}) \cdot (V_2 + \Delta V_{21} + V_1) \xrightarrow{\ solve, P_1\ } -\frac{P_2 \cdot V_2 - (P_2 + \Delta P_{21}) \cdot (V_1 + V_2 + \Delta V_{21})}{V_1}$$

Oscillation

$$P_{12} \cdot V_1 + (P_2 + \Delta P_{21} + \Delta P_{22}) \cdot (V_2 + \Delta V_{21} + \Delta V_{22}) =$$

$$(P_2 + \Delta P_{21}) \cdot (V_2 + \Delta V_{21} + V_1) \xrightarrow{\ solve, P_{12}\ } -\frac{(P_2 + \Delta P_{21} + \Delta P_{22}) \cdot (V_2 + \Delta V_{21} + \Delta V_{22}) - (P_2 + \Delta P_{21}) \cdot (V_1 + V_2 + \Delta V_{21})}{V_1}$$

By parameterizing the volumes, the following equations are determined:

$$V_1 := a \cdot V_2 \qquad \Delta V_{21} := b \cdot V_2 \qquad \Delta V_{22} := c \cdot (V_2 + \Delta V_{21})$$

$$P_1 \cdot V_1 + P_2 \cdot V_2 = (P_2 + \Delta P_{21}) \cdot (V_2 + \Delta V_{21} + V_1) \xrightarrow[\text{simplify}]{\text{solve}, P_1} \frac{a \cdot P_2 + b \cdot P_2}{a} + \frac{\Delta P_{21} \cdot (a + b + 1)}{a}$$

$$P_{12} \cdot V_1 + (P_2 + \Delta P_{21} + \Delta P_{22}) \cdot (V_2 + \Delta V_{21} + \Delta V_{22}) =$$

$$(P_2 + \Delta P_{21}) \cdot (V_2 + \Delta V_{21} + V_1) \xrightarrow[\text{simplify}]{\text{solve}, P_{12}} - \frac{c \cdot P_2 - a \cdot P_2 - a \cdot \Delta P_{21} + c \cdot \Delta P_{21} + b \cdot c \cdot P_2 + b \cdot c \cdot \Delta P_{21}}{a} - \frac{\Delta P_{22} \cdot (b + c + b \cdot c + 1)}{a}$$

In a numerical example of a system 100 comprising a larger cuff 200 (e.g. sized to be worn around a wrist of the subject 300), these equations give:

$$P_2 := 1.0 \; bar \qquad a := 0.1 \qquad V_1 := a \cdot V_2$$

$$\Delta P_{21} := 0.107 \; bar \qquad b := 0.2 \qquad \Delta V_{21} := b \cdot V_2$$

$$\Delta P_{22} := 6.667 \cdot 10^{-3} \; bar \qquad c := 0.02$$

$$P_1 \cdot V_1 + P_2 \cdot V_2 = (P_2 + \Delta P_{21}) \cdot (V_2 + \Delta V_{21} + V_1) \xrightarrow{\text{solve}, P_1} 4.391 \cdot bar$$

$$P_{12} \cdot V_1 + (P_2 + \Delta P_{21} + \Delta P_{22}) \cdot (V_2 + \Delta V_{21} + \Delta V_{22}) =$$

$$(P_2 + \Delta P_{21}) \cdot (V_2 + \Delta V_{21} + V_1) \xrightarrow{\text{solve}, P_{12}} 1.026996 \cdot bar - 11.13667 \cdot c \cdot bar - 11.13667 \cdot b \cdot c \cdot bar = (7.5972 \cdot 10^{-1}) \; bar$$

In another numerical example of a system 100 comprising a smaller cuff 200 (e.g. sized to be worn around a finger of the subject 300), these equations give:

$$P_2 := 1.0 \; bar \qquad a := 1 \qquad V_1 := a \cdot V_2$$

$$\Delta P_{21} := 0.107 \; bar \qquad b := 0.1 \qquad \Delta V_{21} := b \cdot V_2$$

$$\Delta P_{22} := 6.667 \cdot 10^{-3} \; bar \qquad c := 0.01$$

$$P_1 \cdot V_1 + P_2 \cdot V_2 = (P_2 + \Delta P_{21}) \cdot (V_2 + \Delta V_{21} + V_1) \xrightarrow{\text{solve}, P_1} 1.3247 \cdot bar$$

$$P_{12} \cdot V_1 + (P_2 + \Delta P_{21} + \Delta P_{22}) \cdot (V_2 + \Delta V_{21} + \Delta V_{22}) =$$

$$(P_2 + \Delta P_{21}) \cdot (V_2 + \Delta V_{21} + V_1) \xrightarrow{solve, P_{12}} 1.0996663 \cdot bar - 1.113667 \cdot c \cdot bar - 1.113667 \cdot b \cdot c \cdot bar = 1.0874 \ bar$$

The required overpressure in the chamber 102 as a function of the size (or volume) of the chamber 102 is given, as follows:

$$a := 0.1, 0.15..1 \qquad b := 0.1 \qquad b_2 := 0.2$$

$$P_{op1}(a, b, P_2, \Delta P_{21}) := \frac{1}{a} \cdot (P_2 \cdot (a+b) + \Delta P_{21} \cdot (1+a+b))$$

$$P_{op2}(a, b_2, P_2, \Delta P_{21}) := \frac{1}{a} \cdot (P_2 \cdot (a+b_2) + \Delta P_{21} \cdot (1+a+b_2))$$

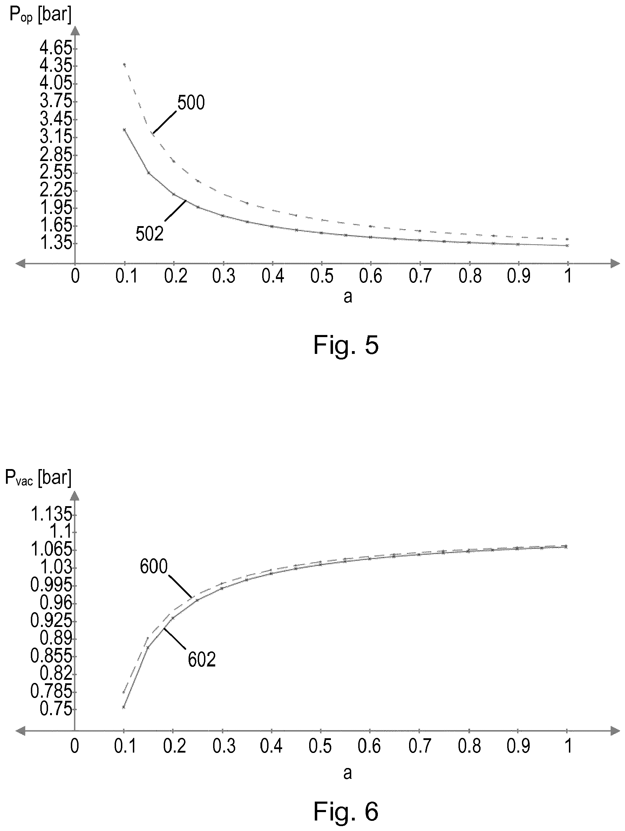

Fig. 5 is a graphical illustration of a pressure as a function of volume ratio for a chamber 102 according to an embodiment. More specifically, Fig. 5 illustrates the required overpressure $P_{op}$ in the chamber 102 as a function of the parameter "a", which is the ratio of the volume $V_1$ of the chamber 102 to the volume $V_2$ of the cuff 200 ($V_1/V_2$). As a function of the parameter "a" (a= $V_2/V_1$), the required overpressure $P_{op}$ in the chamber 102 is plotted for two values of "b", which determines the increase of the volume of the cuff 200 while pressurizing the cuff 200 (to equilibrium). A larger increase ($b_2$ = 20%) 500 requires a higher overpressure than a smaller increase (b= 10) 502. However, in both cases, a clear reduction in the required overpressure with increased size (or volume) of the chamber 102 can be observed.

The resulting minimum pressure (or vacuum) in the chamber 102 as a function of the size (or volume) of the chamber 102 is given, as follows:

$$a := 0.1, 0.15..1 \qquad b := 0.1 \qquad c := 0.02 \qquad b_2 := 0.2$$

$$P_{vac1}(a, b, c, P_2, \Delta P_{21}, \Delta P_{22}) := \frac{1}{a} \cdot ((P_2 + \Delta P_{21}) \cdot (a - c \cdot (1+b)) - \Delta P_{22} \cdot (1+b+c \cdot (1+b)))$$

$$P_{vac2}(a, b_2, c, P_2, \Delta P_{21}, \Delta P_{22}) := \frac{1}{a} \cdot ((P_2 + \Delta P_{21}) \cdot (a - c \cdot (1+b_2)) - \Delta P_{22} \cdot (1+b_2+c \cdot (1+b_2)))$$

Fig. 6 is a graphical illustration of a pressure as a function of volume ratio for a chamber 102 according to another embodiment. More specifically, Fig. 6 illustrates the required overpressure $P_{vac}$ in the chamber 102 (during a peak oscillation) as a function of the parameter "a", which is the ratio of the $V_1$ volume of the chamber 102 to the volume $V_2$ of the cuff 200 ($V_1/V_2$). As a function of the parameter "a" (a= $V_2/V_1$), the resulting pressure $P_{vac}$ in the chamber 102 during a peak oscillation is plotted for two values of "b", which determines the increase of the volume of the cuff 200 while pressurizing it (to equilibrium). A larger increase ($b_2$ = 20) 600 results in a slightly higher pressure than a smaller increase (b= 10%) 602. However, in both cases, a clear increase in the resulting pressure with increased size (volume) of the chamber 102 can be observed.

It can be seen that, if "a" is about 0.36 (i.e. the volume of the chamber 102 is approximately 36% of the volume of the cuff 200), the resulting pressure in the chamber 102 during a peak oscillation is close to the normal or ambient or atmospheric pressure (which can, for example, be assumed to be 1 bar). In such a case, the required overpressure in the chamber 102 is approximately only 2 bar (as can be seen in Fig. 5).

In addition to the system 100 and method 400 described earlier, there is also provided a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein an improved system 100, method 400 and computer program product for use with a wearable cuff 200 in measuring a physiological characteristic of a subject 300.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A system (100) for use with a wearable cuff (200) in measuring a physiological characteristic of a subject (300), the system (100) comprising:

   a chamber (102) configured to hold a fluid (104) at a pressure that is greater than atmospheric pressure; and
   a valve and/or a micro pump (106) configured to couple the chamber (102) to the cuff (200) and operable to control fluid flow between the chamber (102) and the cuff (200) to:

      increase the pressure in the cuff (200) to a predetermined threshold pressure; and
      vary the pressure in the cuff (200) around the predetermined threshold pressure.

2. The system (100) as claimed in claim 1, wherein the valve and/or micro pump (106) is operable to vary the pressure in the cuff (200) by being operable to:

   increase the pressure in the cuff (200) above the predetermined threshold pressure to inflate the cuff (200); and/or
   decrease the pressure in the cuff (200) below the predetermined threshold pressure to deflate the cuff (200).

3. The system (100) as claimed in claim 1 or 2, wherein the predetermined threshold pressure is a pressure at which a difference between the pressure in the chamber (102) and the pressure in the cuff (200) is less than a threshold.

4. The system (100) as claimed in any of the preceding claims, wherein the valve and/or micro pump (106) is operable to vary the pressure in the cuff (200) around the predetermined threshold pressure on the basis of a measured physiological characteristic of the subject (300).

5. The system (100) as claimed in claim 4, wherein the valve and/or micro pump (106) is operable to vary the pressure in the cuff (200) around the predetermined threshold pressure on the basis of oscillations in the measured physiological characteristic of the subject (300).

6. The system (100) as claimed in any of the preceding claims, wherein the chamber (102) is configured to hold a fluid adsorbing material (110).

7. The system (100) as claimed in claim 6, wherein the fluid adsorbing material (110) comprises a zeolite.

8. The system (100) as claimed in any of the preceding claims, wherein a volume of the chamber (102) is less than or equal to a volume of the cuff (200).

9. The system (100) as claimed in any of the preceding claims, wherein the system comprises a further valve connecting the chamber (102) to the atmosphere, wherein the further valve is configured to allow fluid to enter the chamber (102) to increase the pressure in the chamber (102) above atmospheric pressure.

10. The system (100) as claimed in any of the preceding claims, wherein the system comprises the cuff (200).

11. The system (100) as claimed in claim 10, wherein the cuff (200) comprises a first surface (202) configured to contact with the skin of the subject (300) in use and a second surface (204) on which the chamber (102) is positioned, wherein the second surface (204) is opposite the first surface (202).

12. The system (100) as claimed in any of the preceding claims, wherein the chamber (102) and the cuff (200) form a closed system in use.

13. The system (100) as claimed in any of the preceding claims, wherein the system (100) comprises a controller configured to operate the valve and/or micro pump (106) to control the fluid flow between the chamber (102) and the cuff (200).

14. A method of operating a system (100) for use with a wearable cuff (200) in measuring a physiological characteristic of a subject (300), wherein the system comprises a chamber (102) configured to hold a fluid (104) at a pressure that is greater than atmospheric pressure and a valve and/or a micro pump (106) configured to couple the chamber (102) to the cuff (200) and operable to control fluid flow between the chamber (102) and the cuff (200), the method comprising:

increasing the pressure in the cuff (200) to a predetermined threshold pressure; and
varying the pressure in the cuff (200) around the predetermined threshold pressure.

15. A computer program product comprising a computer readable medium, the computer readable medium having a computer readable code embodied therein, the computer readable code being configured such that, on execution by a suitable computer or processor, the computer or processor is caused to perform the method as claimed in claim 14.

100

200

102

106

104

108

300

Fig. 1

Fig. 2

Fig. 3

EP 3 586 731 A1

400

402

404

Fig. 4

Fig. 5

Fig. 6

Europäisches
Patentamt

European
Patent Office

Office européen
des brevets

# EUROPEAN SEARCH REPORT

**Application Number**

EP 18 17 9799

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/330112 A1 (LAMEGO MARCELO [US] ET AL) 27 December 2012 (2012-12-27) * abstract; figures 1-7 * * paragraph * ----- | 1-15 | INV. A61B5/022 A61B5/0225 A61B5/0235 |
| X | US 2010/249616 A1 (DONEHOO ROBERT F [US] ET AL) 30 September 2010 (2010-09-30) * abstract; figures 1-7 * * paragraphs [0006] - [0009], [0018] - [0043] * ----- | 1-15 | |
| X A | US 2006/217617 A1 (WACHTENBERG EYAL [IL]) 28 September 2006 (2006-09-28) * abstract; figures 1-7 * * paragraphs [0089] - [0119] * ----- | 1-11, 13-15 12 | |
| X A | US 4 627 440 A (RAMSEY III MAYNARD [US] ET AL) 9 December 1986 (1986-12-09) * abstract; figures 1-3 * * column 3, line 12 - column 6, line 37 * ----- | 1-11, 13-15 12 | |
| | | | **TECHNICAL FIELDS SEARCHED (IPC)** A61B |

The present search report has been drawn up for all claims

| Place of search | Date of completion of the search | Examiner |
|---|---|---|
| The Hague | 30 November 2018 | Carta, Riccardo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 17 9799

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2018

| Patent document cited in search report | | Publication date | Patent family member(s) | | Publication date |
|---|---|---|---|---|---|
| US 2012330112 | A1 | 27-12-2012 | US 2012330112 A1<br>US 2017196470 A1 | | 27-12-2012<br>13-07-2017 |
| US 2010249616 | A1 | 30-09-2010 | NONE | | |
| US 2006217617 | A1 | 28-09-2006 | CA 2524406 A1<br>CN 1784171 A<br>EP 1622509 A1<br>JP 2007533357 A<br>KR 20060017515 A<br>US 2006217617 A1<br>WO 2004100783 A1 | | 25-11-2004<br>07-06-2006<br>08-02-2006<br>22-11-2007<br>23-02-2006<br>28-09-2006<br>25-11-2004 |
| US 4627440 | A | 09-12-1986 | AT 80278 T<br>AU 583637 B2<br>BR 8603129 A<br>DE 3686678 D1<br>DE 3686678 T2<br>DK 321386 A<br>EP 0207805 A2<br>FI 862855 A<br>JP S628734 A<br>JP H0527417 B2<br>MX 165075 B<br>NO 862721 A<br>US 4627440 A<br>ZA 8605003 B | | 15-09-1992<br>04-05-1989<br>17-02-1987<br>15-10-1992<br>28-01-1993<br>06-01-1987<br>07-01-1987<br>06-01-1987<br>16-01-1987<br>21-04-1993<br>21-10-1992<br>06-01-1987<br>09-12-1986<br>24-02-1988 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100106029 A